# FASCICULE DE BREVET EUROPEEN

(11) **EP 1 837 013 B1**
(45) Date de publication et mention de la délivrance du brevet: **19.04.2017**
(21) Numéro de dépôt: 07103645.3
(22) Date de dépôt: 06.03.2007
(51) Int. Cl.: A61Q 19/00, A61K 8/46, A61K 8/19

(54) **Composition comprenant de l'hydroxyapatite et un sel de calcium pour renforcer la fonction barrière de la peau et/ou des semi-muqueuses.**
Zusammensetzung, die Hydroxyapatit und ein Kalziumsalz umfasst, zum Verstärken der Barrierefunktion der Haut und/oder der Halbschleimhäute
Composition comprising hydroxyapatite and a calcium salt for reinforcing the barrier function of the skin and/or semi-mucus membranes.

(30) Priorité: 23.03.2006 FR 0651008
(43) Date de publication de la demande: 26.09.2007
(73) Titulaire: L'Oréal, 75008 Paris (FR)
(72) Inventeur: Mackowiak, Vanessa, 92160 Antony (FR)
(74) Mandataire: Brohmi, Karim

(56) Documents cités:
- EP-A1- 1 293 193
- FR-A- 2 812 194
- FR-A1- 2 594 130
- FR-A1- 2 870 455
- US-A- 4 749 719
- US-A- 5 122 418
- US-A- 6 120 782
- US-A1- 2005 089 516

## Description

L'invention concerne une composition de soin, en particulier des peaux matures, voire des peaux très matures, destinée à améliorer et/ou renforcer la fonction barrière de la peau et/ou des semi-muqueuses.

Par 'semi-muqueuses', on entend notamment les lèvres.

Par 'peaux matures' selon l'invention, on entend notamment des peaux de sujets ayant au moins 40 ans.
Par 'peaux très matures' selon l'invention, on entend notamment des peaux de sujets ayant au moins 50 ans, en particulier au moins 60 ans, voire 65 ans.

L'invention concerne notamment une composition comprenant, dans un milieu physiologiquement acceptable, au moins de l'hydroxyapatite au moins en partie sous forme libre, et au moins un sel de calcium, le pantetheine sulfonate de calcium.
Elle se rapporte également à un procédé cosmétique de soin, notamment des peaux matures voire très matures, mettant en oeuvre cette association et visant notamment à améliorer la fonction barrière de la peau et/ou des semi-muqueuses et favoriser ainsi leur protection contre la déperdition en eau et les agressions extérieures.

La peau humaine est constituée de deux compartiments, à savoir un compartiment profond, le derme et un compartiment superficiel, l'épiderme.

L'épiderme est en contact avec l'environnement extérieur. Un de ses rôles consiste à protéger l'organisme de la déshydratation et des agressions extérieures, notamment liées à des facteurs environnementaux de type agents irritants ou polluants (détergents, pollution, fumée de cigarettes...), sollicitations mécaniques (frottements, abrasion, rasage, lavage fréquent...), déséquilibres thermiques ou climatiques (froid, vent, sécheresse, radiations UV...), xénobiotiques (micro-organismes, allergènes...), traitements chimiques cosmétiques ou dermatologiques (peeling, traitement anti-acné...), ou par des facteurs physiologiques (âge, stress...).

Il est composé principalement de trois types de cellules qui sont les kératinocytes, très majoritaires, les mélanocytes et les cellules de Langerhans, qui sont délimitées par une structure lipidique intercellulaire. Cette structure lipidique ainsi que la cohésion des cellules épidermiques participent aux échanges en eau de la peau et à la fonction protectrice ou 'fonction barrière' de la peau, d'une part vis-à-vis des déperditions en eau (perte insensible en eau) et d'autre part vis-à-vis des agressions extérieures.

La fonction barrière de la peau et/ou des semi-muqueuses peut être altérée notamment par des facteurs physiologiques internes tels que l'âge, le stress, des changements hormonaux et/ou par des facteurs environnementaux tels que des déséquilibres thermiques ou climatiques, des xénobiotiques, des agents irritants, des sollicitations mécaniques de type frottements, lavage, rasage....

La peau et/ou les semi-muqueuses se fragilisent, deviennent plus sèches, plus sujettes aux crevasses et aux gerçures, moins résistantes aux frictions mécaniques et plus perméables à l'environnement extérieur et aux bactéries.

Sur la peau et/ou les lèvres, l'apparition d'une sécheresse et/ou de microgerçures peut avoir pour conséquence une moins bonne homogénéité du maquillage appliqué sur celles-ci (ex : fond de teint, rouge à lèvres), ce qui peut ne pas être esthétique.

L'altération de la fonction barrière de la peau et/ou des semi-muqueuses s'accentue avec l'âge notamment après 40 ans, en particulier après 50 ans et c'est encore plus vrai après 60-65 ans, aussi bien chez les femmes que les hommes, et en particulier chez les femmes.

On sait par exemple qu'une chute hormonale au cours de la ménopause entraîne une baisse de la teneur en lipides cutanés. La peau devient alors plus sèche, plus fragile et plus facilement sujette aux crevasses et aux gerçures.
On assiste à une désorganisation générale de la structure cutanée liée à une diminution de la cohésion entre les cellules, tant au niveau de l'épiderme que du derme.

Outre les peaux âgées et en particulier les peaux matures, voire très matures, d'autres types de peaux ont une fonction barrière cutanée diminuée ; il s'agit notamment :
- des peaux sensibles,
- des peaux sèches dont le film hydrolipidique protecteur est modifié ou altéré.

Pour améliorer et/ou renforcer la fonction barrière de la peau et/ou des semi-muqueuses, une solution traditionnelle consiste à apporter en permanence des agents externes lipidiques (ex : corps gras, céramides) et/ou des agents favorisant la synthèse de lipides épidermiques (ex : vitamine C et ses dérivés) et/ou des agents émollients ou hydratants.

Mais il subsiste toujours le besoin de trouver de nouvelles compositions permettant d'améliorer la cohésion cellulaire et/ou renforcer la fonction barrière de la peau et/ou des semi-muqueuses, en particulier des compositions adaptées au soin des peaux matures, voire des peaux très matures.

La Demanderesse vient de montrer que l'hydroxyapatite, en particulier au moins en partie sous forme libre et notamment sous forme de particules de 1 à 10µm associée à au moins un sel de calcium permettait de répondre à ce besoin.
En particulier, elle a montré que le pantetheine sulfonate de calcium permettait de potentialiser et/ou augmenter l'effet de l'hydroxyapatite sur l'amélioration de la fonction barrière de la peau.
Cette association permet en outre de diminuer la quantité d'hydroxyapatite nécessaire pour obtenir l'effet recherché sur la peau et/ou des semi-muqueuses, à savoir un effet sur l'amélioration de la fonction barrière.

L'utilisation de l'hydroxyapatite sous forme de particules poreuses de 1 à 10µm dans des compositions cosmétiques hydratantes ou nettoyantes est connue de la demande WO96/41611: l'hydroxyapatite sous forme de particules poreuses associée à des agents hydratants et antimicrobiens, sert de vecteur et permet notamment de favoriser l'absorption, le transport et la libération de ces actifs sur la peau.

Mais à la connaissance de la Demanderesse, il n'avait jamais été suggéré jusqu'ici d'associer l'hydroxyapatite, notamment au moins en partie sous forme libre, à un sel de calcium pour potentialiser et/ou augmenter leur effet, en particulier pour améliorer la cohésion des cellules et/ou renforcer la fonction barrière de la peau et/ou des semi-muqueuses.

Par 'hydroxyapatite au moins en partie sous forme libre' utilisée selon l'invention, on entend de l'hydroxyapatite au moins en partie non associée et/ou non complexée à une autre poudre de type organique, minérale ou métallique, par opposition à la forme d'hydroxyapatite constitutive d'une poudre composite au sens de la demande FR 2594130.
Cette demande décrit une poudre composite dans laquelle une poudre organique ou minérale formant le noyau est recouverte de façon sensiblement complète par un autre type de poudre, tel qu'une poudre d'hydroxyapatite. Cette forme composite est destinée à améliorer les caractéristiques superficielles de la poudre, en particulier sa capacité à absorber les déchets du sébum et son pouvoir couvrant, par rapport à de l'hydroxyapatite au moins en partie sous forme libre.

L'hydroxyapatite utilisée selon la présente invention est de préférence de l'hydroxyapatite au moins en partie sous forme libre, en particulier au moins à 50% sous forme libre, de préférence au moins à 70%, plus préférentiellement au moins à 90% sous forme libre, et mieux, à 100% sous forme libre.

L'invention concerne donc notamment une composition, comprenant, dans un milieu physiologiquement acceptable, au moins de l'hydroxyapatite au moins en partie sous forme libre et au moins le pantetheine sulfonate de calcium.

### Hydroxyapatite

L'hydroxyapatite est un phosphate de calcium avec un rapport molaire calcium/phosphore allant de 0,5 à 20 ayant une structure d'apatite (Fragrance Journal, pages 144 à 148, Janvier 1999).

Elle est présente à l'état naturel dans la substance fondamentale de la matrice extracellulaire osseuse et les dents. Elle se présente sous forme cristallisée, c'est-à-dire avec une séquence ordonnée des atomes dans l'espace. Les cristaux d'hydroxyapatite sont visibles au microscope électronique entre les fibres de collagène ou à l'intérieur de celui-ci et ont une allure d'aiguilles allongées.

L'hydroxyapatite a une formule brute [Ca₁₀(PO₄)₆(OH)₂] ou 3Ca₃(PO₄)₂ Ca(OH)₂.

L'hydroxyapatite peut être d'origine naturelle ou synthétique.

Elle peut notamment être obtenue par voie de synthèse par réaction entre un hydroxyde de calcium et un acide phosphorique.

En particulier, l'hydroxyapatite utilisée selon l'invention est au moins en partie sous forme libre, en particulier au moins à 50% sous forme libre, de préférence au moins à 70%, plus préférentiellement au moins à 90% sous forme libre, et mieux, à 100% sous forme libre.

De préférence, l'hydroxyapatite utilisée dans la composition de l'invention est présente sous forme de particules ayant une taille moyenne en nombre inférieure ou égale à 50 µm, notamment allant de 0,1 µm à 50 µm, de préférence inférieure ou égale à 20 µm, notamment allant de 0,1 µm à 20 µm, et préférentiellement inférieure ou égale à 10 µm, notamment allant de 0,1 µm à 10 µm.

De préférence, l'hydroxyapatite utilisée selon l'invention se présente sous la forme de particules ayant une taille moyenne en nombre allant de 1 à 10µm, de préférence de 2 à 6µm et encore plus préférentiellement une taille de 4µm.

Par 'taille moyenne en nombre', on entend le diamètre moyen d'une population de particules. Ce diamètre moyen peut être déterminé par microscopie électronique à transmission ou à partir de la mesure de la surface spécifique par la méthode BET ou bien par l'intermédiaire d'un granulomètre laser.

L'hydoxyapatite peut représenter de 5 % à 99 % en poids du poids total des particules.

De préférence, l'hydroxyapatite représente au moins 50% en poids du poids total des particules, mieux encore au moins 70 % en poids du poids total des particules, et encore plus préférentiellement au moins 90 % en poids du poids total des particules.

En particulier, on utilisera de l'hydroxyapatite sous forme de particules en hydroxyapatite substantiellement pur, c'est-à-dire comprenant au moins 99% en poids d'hydroxyapatite par rapport au poids total des particules.

De telles particules d'hydroxyapatite sont décrites dans la demande WO96/41611.
Elles sont obtenues par agglomération de cristaux d'hydroxyapatite de taille moyenne allant de 0.05 à 0.10µm, formant des particules sphériques qui sont ensuite frittées à haute température pour l'obtention de particules sphériques poreuses qui sont mécaniquement, physiquement et chimiquement stables. Le diamètre moyen en nombre de ces particules va de 1 à 10µm, de préférence de 2 à 6µm.
Ces particules permettent d'absorber, transporter et ultérieurement libérer les actifs de la composition sur la peau.
De telles particules poreuses sphériques sont par exemple commercialisées sous la dénomination Hydroxysomes™ par la société Laboratory Skin Care (LSC).

L'hydroxyapatite selon l'invention est utilisée en une quantité suffisante en association avec le sel de calcium pour obtenir l'effet bénéfique recherché sur la peau et/ou les semi-muqueuses, en particulier un effet sur l'amélioration de la fonction barrière.
Cette quantité peut être évaluée par exemple sur un modèle de peau tel que décrit dans les exemples ci-après.
A titre d'exemple, on utilisera une quantité allant de 0,001 % à 10% en poids par rapport au poids total de la composition, de préférence de 0,01 à 5% en poids et encore plus préférentiellement de 0,05 à 1% en poids par rapport au poids total de la composition.

### Sels de calcium

Le sel organique préféré est le pantetheine sulfonate de calcium de formule 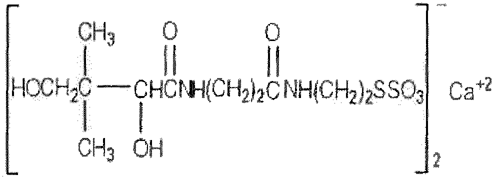 autrement nommé calcium pantetheine sulfonate ou Bis (N-pantothenyl-beta-aminoethyl) thiosulfate de calcium.

On utilisera par exemple le Calcium D-Pantéthéine-S-Sulfonate 70, autrement nommé Bis(N-pantothenyl-beta-aminoethyl) thiosulfate de calcium sous forme de solution aqueuse à 70% de matière active commercialisé par la société SOGO PHARMACEUTICAL.

La quantité de pantetheine sulfonate de calcium utilisable dans la composition de l'invention en association avec l'hydroxyapatite est fonction de l'effet bénéfique recherché sur la peau et/ou les semi-muqueuses, en particulier fonction de l'effet recherché sur l'amélioration de la fonction barrière.

A titre d'exemple, la quantité présente dans la composition selon l'invention ira de 0,01 à 10% en poids par rapport au poids total de la composition, de préférence de 0,01 à 5% en poids par rapport au poids total de la composition et encore plus préférentiellement de 0,05 à 1% en poids par rapport au poids total de la composition.

En particulier, l'hydroxyapatite et le pantetheine sulfonate de clacium sont présents dans la composition dans un rapport de concentration allant de 10 :1 à 1 :10, de préférence de 10 :1 à 1 :5.

En particulier, on utilisera l'hydroxyapatite et le pantetheine sulfonate de calcium dans un rapport de concentration allant de 7 :1 à 3 :1, et encore plus préférentiellement dans un rapport de concentration de 5 :1.

La composition selon l'invention peut également contenir en outre au moins un actif à effet complémentaire à l'association selon l'invention, tels qu'au moins un composé choisi parmi les agents desquamants et/ou hydratants ; les agents stimulant la synthèse de macromolécules du derme et/ou prévenant leur dégradation ; les agents stimulant la prolifération des fibroblastes ou des kératinocytes et/ou la différenciation des kératinocytes ; les agents favorisant la synthèse des lipides épidermiques ; les lipides épidermiques, en particulier les céramides ; les agents anti-oxydants et anti-radicalaires, et leurs mélanges.

### Agents desquamants et/ou hydratants

Comme exemples d'agents desquamants et/ou hydratants, on peut citer par exemple les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique, et leurs mélanges ; l'urée ; l'acide gentisique ; les oligofucoses ; l'acide cinnamique ; l'extrait de Saphora japonica ; le resvératrol et certains dérivés d'acide jasmonique .
Un agent desquamant préféré est l'acide n-octanoyl 5-salicylique.

Ces agents desquamants et/ou hydratants peuvent représenter de 0,01% à 5%, et de préférence de 0,1 à 1%, du poids total de la composition selon l'invention.

### Agents stimulant la synthèse de macromolécules dermiques et/ou empêchant leur dégradation

Parmi les actifs stimulant les macromolécules du derme ou empêchant leur dégradation, on peut citer ceux qui agissent :
- soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; Palmitoyl pentapeptide- 3 ou Matrixyl ; les peptides extraits de végétaux, tels que l'hydrolysat de soja commercialisé par la société COLETICA sous la dénomination commerciale Phytokine^{®} ; et les hormones végétales telles que les auxines et les lignanes ;

- soit sur l'inhibition de la dégradation du collagène, en particulier des agents agissant sur l'inhibition des métalloprotéinases (MMP) telles que plus particulièrement les MMP 1, 2, 3, 9 . On peut citer : les rétinoïdes et dérivés, les oligopeptides et les lipopeptides, les lipoaminoacides, l'extrait de malt commercialisé par la société COLETICA sous la dénomination commerciale Collalift^{®} ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB^{®}), de trèfle rouge, de lin, de kakkon ou de sauge ; la DIPALMITOYL HYDROXYPROLINE commercialisé par Seppic sous le nom SEPILIFT DPHP^{®} : Baccharis genistelloide ou Baccharine commercialisé par SILAB ;
- soit sur la synthèse de molécules appartenent à la famille des élastines (élastine et fibrilline), tels que : le retinol et dérivés ; l'extrait de *Saccharomyces Cerivisiae* commercialisé par la société LSN sous la dénomination commerciale Cytovitin^{®} ; et l'extrait d'algue *Macrocystis pyrifera* commercialisé par la société SECMA sous la dénomination commerciale Kelpadelie^{®} ; et les composés N-acylaminoamides décrits dans la demande WO 01/94381 tels que l'acide {2-[acétyl-(3-trifluorométhyl-phényl)-amino]-3-méthyl-butyrylamino} acétique) autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine ;
- soit sur l'inhibition de la dégradation de l'élastine tels que l'extrait peptidique de graines de *Pisum sativum* commercialisé par la société LSN sous la dénomination commerciale Parelastyl^{®} ; les héparinoïdes ; et les composés N-acylaminoamides décrits dans la demande WO 01/94381 tels que l'acide {2-[acétyl-(3-trifluorométhyl-phényl)-amino]-3-méthyl-butyrylamino} acétique) autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine;

Un agent préféré agissant sur la synthèse de collagène est un hydrolysat de soja, tel que celui commercialisé par la société COLETICA sous la dénomination commerciale Phytokine^{®}.
Un agent préféré agissant sur la synthèse de molécules appartenant à la famille des élastines est un extrait de *Saccharomyces Cerivisiae,* tel que celui commercialisé par la société LSN sous la dénomination commerciale Cytovitin^{®.}

Ces agents augmentant la synthèse de macromolécules du derme et/ou prévenant leur dégradation peuvent représenter de 0,01% à 5%, et de préférence de 0,1 à 1%, du poids total de la composition selon l'invention.

### Agent stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8^{®} ou commercialisé par la société SILAB sous la dénomination commerciale Raffermine^{®}) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; l'adénosine ; le phloroglucinol ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par un extrait peptidique de lupin tel que celui commercialisé par la société SILAB sous la dénomination commerciale Structurine^{®}; le beta-sitosteryl sulfate de sodium tel que celui commercialisé par la société SEPORGA sous la dénomination commerciale Phytocohésine^{®} ; et un extrait hydrosoluble de maïs tel que celui commercialisé par la société SOLABIA sous la dénomination commerciale Phytovityl^{®} ; un extrait peptidique de *Voandzeia substerranea* tel que celui commercialisé par la société Laboratoires Sérobiologiques sous la dénomination commerciale Filladyn LS 9397^{®} ; et les lignanes tels que le sécoisolaricirésinol.

### Agents favorisant la synthèse de lipides épidermiques

On peut citer par exemple la vitamine C et ses dérivés, en particulier son dérivé 2-O-α-D glucopyranosyl d'acide L-ascorbique (ou glucoside d'ascorbyle) ; l'acide cinnamique et ses dérivés ; les auxines.

### Galénique

La composition selon l'invention est adaptée à une application par voie topique sur la peau et/ou les semi-muqueuses ou adaptée à une administration par voie orale.
De préférence, il s'agira d'une composition adaptée à une application topique sur la peau et/ou les lèvres.
Cette composition peut être une composition de soin ou une composition de maquillage. De préférence, il s'agira d'une composition de soin.

La composition pourra être une composition cosmétique ou dermatologique. De préférence, il s'agira d'une composition cosmétique, en particulier d'une composition de soin de la peau et/ou des lèvres.

Pour une administration par voie orale, en particulier en 'cosmétique orale', elle peut se présenter notamment sous forme de capsules, de gélules, dragées, de granulés, de pâte à mâcher, de gels, de sirops buvables,de comprimés ou de toute autre forme connue de l'homme du métier. En particulier, l'association des actifs selon l'invention peut être incorporée dans toutes formes de compléments alimentaires ou d'aliments enrichis, par exemple des barres alimentaires, ou des poudres compactées ou non. Les poudres peuvent être diluables à l'eau, dans du soda, des produits laitiers ou dérivés du soja, ou être incorporées dans des barres alimentaires. Les actifs peuvent être formulés avec les excipients et composants usuels pour de tels compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire. Les agents de formulation et excipients pour composition orale, et notamment pour compléments alimentaires sont connus dans ce domaine et ne font pas ici l'objet d'une description détaillée.

La composition selon l'invention comprend un milieu physiologiquement acceptable, c'est-à-dire compatible avec la peau et/ou ses phanères. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

Pour une application topique sur la peau et/ou les semi-muqueuses, la composition selon l'invention peut se présenter sous toutes les formes galéniques classiquement utilisées pour une application topique et notamment sous forme de dispersions du type lotion ou gel aqueux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème, gel, sérum, stick, masque, ou encore d'émulsions multiples (E/H/E ou H/E/H), d'aérosols, de microémulsions, de dispersions vésiculaires de type ionique et/ou non ionique, ou de dispersions cire/phase aqueuse. Ces compositions sont préparées selon les méthodes usuelles.

Selon un mode préféré, la composition selon l'invention ne sera pas une poudre.

Selon un autre mode préféré, la composition selon l'invention ne sera pas une composition anhydre.

On peut également envisager une composition sous forme de mousse ou encore sous forme de spray ou d'aérosol comprenant alors un agent propulseur sous pression.

Selon un mode préféré de réalisation de l'invention, la composition se présente sous forme d'une émulsion H/E (ex : crème) ou E/H (ex : sérum) ou d'un gel de type aqueux (ex : lotion).

Lorsque la composition est une émulsion, la proportion de la phase grasse de la composition considérée peut aller par exemple de 5 à 80 % en poids, et notamment de 5 à 50 % en poids par rapport au poids total de la composition.

La phase aqueuse peut être constituée essentiellement d'eau ou comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, et les aldéhydes en C₂-C₄.

Cette phase aqueuse (eau et éventuellement le solvant organique miscible à l'eau) peut être présente dans la composition de base en une teneur allant de 1 % à 95 % en poids, notamment allant de 3 % à 80 % en poids, et en particulier allant de 5 % à 60 %, en poids par rapport au poids total de la composition de base.

La phase grasse de la composition peut contenir des composés gras ou huileux, et éventuellement des cires, des gommes, des corps gras pâteux d'origine végétale, animale, minérale ou de synthèse, siliconé ou non.

Comme huiles utilisables dans la composition selon l'invention, on peut citer :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone ou encore, par exemple, les huiles végétales telles que l'huile d'amande d'abricot et l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone ;
- les hydrocarbures linéaires ou ramifiés, d'origine minérale ou synthétique, tels que les huiles de paraffine, volatiles ou non, et leurs dérivés, l'isohexadecane, l'isododecane, la vaseline, les polydécènes, le polyisobutène hydrogéné tel que l'huile de Parléam® ;
- des huiles essentielles naturelles ou synthétiques ;
- les alcools gras ramifiés ayant de 8 à 26 atomes de carbone, comme l'octyldodécanol ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que le cyclohexasiloxane et le cyclopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; et
- leurs mélanges.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les alcools gras linéaires tels que les alcools cétylique et/ou stéarylique ; les corps gras pâteux comme la lanoline ; les cires ; et les gommes telles que les gommes de silicone (diméthiconol).

Ces corps gras peuvent être choisis de manière variée par l'homme du métier afin de préparer une composition ayant les propriétés, par exemple de consistance ou de texture, souhaitées.

Cette composition peut en outre contenir divers adjuvants couramment utilisés dans le domaine cosmétique, tels que des émulsionnants dont l'acide stéarique, les esters d'acides gras et de polyéthylène glycol, les esters d'acide gras et de sorbitane éventuellement polyoxyéthylénés, les alcools gras polyoxyéthylénés et les esters ou éthers d'acide gras et de sucres tel que le sucrose ou le glucose ; des conservateurs ; des séquestrants ; des parfums ; et des épaississants et/ou des gélifiants, en particulier les polyacrylamides, les homo- et copolymères acryliques et les homo- et copolymères d'acide acrylamido méthylpropane sulfonique ; des charges ; et des agents photoprotecteurs actifs dans l'UVA et/ou l'UVB, autrement nommés filtres UV.

Comme charges, on peut citer par exemple, les particules de polyamide (Nylon) sous forme sphérique ou sous forme de microfibres ; les microsphères de polyméthacrylate de méthyle ; les poudres de copolymère éthylène-acrylate ; les poudres expansées telles que les microsphères creuses et notamment, les microsphères formées d'un terpolymère de chlorure de vinylidène, d'acrylonitrile et de méthacrylate et commercialisées sous la dénomination EXPANCEL ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinique ; les microbilles de résine de silicone telles que celles commercialisées sous la dénomination TOSPEARL par la société Toshiba Silicone ; la silice ; les oxydes métalliques tels que le dioxyde de titane ou l'oxyde de zinc ; le mica ; les particules hémisphériques creuses de silicone telles que le NLK506 commercialisé par la société Takemoto Oil and Fat; et leurs mélanges.

En particulier, la composition selon l'invention comprendra au moins un filtre UV.

Comme filtres UV ou agents photoprotecteurs actifs dans l'UVA et/ou l'UVB utilisables dans la composition de l'invention, on peut citer notamment les agents photoprotecteurs organiques ou inorganiques.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 ; et les polymères filtres et silicones filtres tels que ceux décrits notamment dans la demande WO-93/04665 ; les dimères dérivés d'α-alkylstyrène tels que ceux décrits dans la demande de brevet DE19855649.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans la composition selon l'invention dans des proportions allant de 0,1 à 20 % en poids par rapport au poids total de la composition, et de préférence allant de 0,2 à 15 % en poids par rapport au poids total de la composition.

Bien entendu, l'homme du métier veillera à choisir ce ou ces éventuels composés additionnels et/ou leur quantité de manière telle que les propriétés de la composition selon l'invention ne soient pas, ou substantiellement pas, altérées par l'adjonction envisagée.

En particulier, la composition selon l'invention est sous une forme adaptée à une application topique sur la peau et/ou les semi-muqueuses, telle que décrite précédemment.

L'application topique de la composition selon l'invention se fait selon les techniques habituelles, par exemple par application de crèmes, de gels, de sérums, de lotions, de sticks, de masques sur la peau et/ou les lèvres destinées à être traitées, en particulier sur les lèvres ou sur la peau du corps, du visage et/ou du cou.

Selon une alternative, la composition selon l'invention peut être sous une forme adaptée à une administration par voie orale, telle que décrite précédemment.

L'invention porte également sur un procédé cosmétique de soin de la peau et/ou des semi-muqueuses, comprenant l'administration par voie orale et/ou l'application sur la peau, *via* au moins une composition, de l'association d'au moins de l'hydroxyapatite au moins en partie sous forme libre et d'au moins le pantetheine sulfonate de calcium.

Selon un premier procédé de l'invention, les deux actifs sont conditionnés dans la même composition, sous une forme adaptée à une administration par voie orale ou sous une forme adaptée à une application topique sur la peau et/ou les semi-muqueuses.

Lorsque les deux actifs sont conditionnés dans une composition topique, celle-ci comprend de préférence au moins de l'hydroxyapatite au moins en partie sous forme libre et au moins un sel de calcium.

Lorsque les deux actifs sont conditionnés dans une composition orale, celle-ci comprend au moins de l'hydroxyapatite et au moins un sel de calcium.

L'invention concerne donc un procédé cosmétique de soin de la peau et/ou des semi-muqueuses comprenant l'administration par voie orale ou l'application topique sur la peau d'une composition telle que définie précédemment.

Selon une autre procédé de l'invention, les deux actifs sont conditionnés dans deux compositions distinctes, qui peuvent être respectivement sous une forme adaptée à une application topique ou sous une forme adaptée à une administration par voie orale ou encore pour l'une des compositions sous une forme pour application topique et pour l'autre composition sous une forme pour administration orale (kit mixte).

Les compositions distinctes selon ce procédé comprennent respectivement au moins de l'hydroxyapatite et au moins un sel de calcium.

Lorsque les deux compositions sont sous une forme adaptée à une application topique, elles peuvent être conditionnées soit dans deux dispositifs d'application distincts soit dans deux compartiments d'un même dispositif d'application, permettant soit une distribution simultanée des deux compositions au moment de l'application sur la peau soit une distribution et une libération séparée des deux compositions.

L'invention concerne donc également un procédé cosmétique de soin de la peau et/ou des semi-muqueuses comprenant l'administration par voie orale et/ou l'application topique sur la peau, *via* au moins deux compositions, de l'association d'au moins de l'hydroxyapatite au moins en partie sous forme libre et d'au moins le pantetheine sulfonate de calcium.

En particulier, le procédé selon l'invention est destiné à améliorer l'hydratation et/ou le confort de la peau et/ou renforcer la protection de la peau et/ou des semi-muqueses contre les agressions extérieures.

Il vise également à prévenir et/ou diminuer la formation des crevasses ou gerçures sur la peau et/ou les semi-muqueuses.

Selon un mode préféré, la ou les compositions selon l'invention sont administrées et/ou appliquées sur des sujets ayant une peau mature, voire très mature.
En particulier, la ou les compositions sont destinées à des sujets ayant au moins 50, voire au moins 60-65 ans.

La ou les compositions selon l'invention peuvent également être administrées et/ou appliquées avantageusement sur les peaux sensibles et/ou les peaux sèches.

La ou les compositions peuvent être administrées et/ou appliquées quotidiennement sur la peau et/ou les semi-muqueuses, le matin et/ou le soir.

L'invention porte encore sur l'utilisation de l'association d'au moins de l'hydroxyapatite au moins en partie sous forme libre et d'au moins le pantetheine sulfonate de calcium, comme association destinée à améliorer et/ou renforcer la cohésion cellulaire et/ou l'intégrité de la peau et/ou des semi-muqueuses et/ou améliorer et/ou renforcer la fonction barrière de la peau et/ou des semi-muqueuses.

En particulier, ladite association et/ou ladite composition selon l'invention est destinée à prévenir et/ou diminuer la fragilisation de la peau et/ou des semi-muqueuses et/ou protéger la peau et/ou les semi-muqueuses contre les déperditions en eau et/ou les agressions extérieures, en particulier :
o contre les déperditions en eau et ainsi prévenir, améliorer et/ou remédier aux signes extérieurs de sécheresse cutanée liés à un déficit de la fonction barrière cutanée ; et/ou
o contre les agressions extérieures, notamment liées à des facteurs environnementaux (de type agents irritants ou polluants (détergents, pollution, fumée de cigarettes...), sollicitations mécaniques (frottements, abrasion, rasage, lavage fréquent des mains), déséquilibres thermiques ou climatiques (froid, sécheresse, radiations), xénobiotiques (micro-organismes, allergènes), traitements chimiques cosmétiques ou dermatologiques (peeling, traitement anti-acné...), ou par des facteurs physiologiques (âge, stress...); et/ou rendre la peau et/ou les semi-muqueuses plus résistantes aux agressions extérieures.

Selon un autre mode, ladite association et/ou ladite composition selon l'invention est destinée à améliorer le confort de la peau, en particulier prévenir et/ou diminuer les tiraillements, les picotements.

Selon un autre aspect de l'invention, ladite association et/ou ladite composition selon l'invention est destinée à améliorer l'aspect de surface de la peau et/ou des lèvres, en particulier la rugosité et/ou les irrégularités de relief.

Selon un autre aspect de l'invention, ladite association et/ou ladite composition selon l'invention est destinée à limiter la formation de gerçures ou crevasses sur les lèvres, les mains, le visage ou le corps et favoriser ainsi notamment une meilleure homogénéité d'une composition de maquillage appliquée ultérieurement sur la peau et/ou les lèvres.

L'hydroxyapatite et le sel de calcium peuvent être conditionnés dans une même composition ou dans deux compositions distinctes.

Ces deux compositions distinctes complémentaires peuvent être présentes dans un kit ou dans deux compartiments d'un même dispositif d'application pour une libération simultanée ou séquentielle desdites compositions, lesdites compositions pouvant être chacune sous une forme adaptée à une application topique ou sous une forme adaptée à une administration par voie orale.

Des exemples de compositions adaptées respectivement à une application topique ou à une administration par voie orale sont décrites précédemment.

Selon un mode préféré, on utilisera une hydroxyapatite au moins en partie sous forme libre.

L'invention sera maintenant illustrée par les exemples non limitatifs suivants. Dans ces exemples, les quantités sont indiquées en pourcentage pondéral.

### EXEMPLES

### Exemple 1 : Effet de l'association d'hydroxyapatite et d'un sel de calcium sur l'amélioration et/ou le renforcement de la fonction barrière

L'hydroxyapatite sous la forme de particules commercialisées sous la dénomination Hydroxysomes™ par LSC et leur association avec un calcium D-pantéthéine sulfonate commercialisé par SOGO Pharmaceutical ont été testées sur un modèle *in vitro* permettant d'évaluer l'effet global de cette association sur la fonction barrière de la peau.

Ce test consiste à appliquer les actifs à tester sur des épidermes reconstruits Episkin™ différenciés à J13, puis à appliquer après 5 jours d'incubation, de la caféine marquée radioactivement et à suivre la diffusion de la caféine marquée à travers les épidermes reconstruits.

La Vitamine C, connue pour améliorer la fonction barrière épidermique *via* une stimulation de la synthèse des lipides épidermiques, est utilisée en tant que molécule de référence pour cet essai (contrôle positif).
On utilise l'eau comme témoin négatif.

Des épidermes Episkin™ différenciés (J13) ont été placés en plaques 12 puits contenant 2 ml de milieu de différenciation et ont été pré-cultivés à 37°C et 5 % CO₂ pendant 24 h. Après incubation, les épidermes ont été traités en topique avec 100 µl de chaque concentration de produit à tester ou du produit de référence (vitamine C).
Des épidermes témoins ont été traités, en topique, avec 100 µl d'eau.

Les épidermes ont ensuite été incubés à 37°C et 5 % CO₂ pendant 5 jours, avec changement de milieu et nouvelle application desdits actifs au bout de 2 jours.

Les traitements ont été réalisés en quintuplicata (n=5).
Quatre épidermes ont été utilisés pour les passages de la caféine et un épiderme «contrôlé» a été conservé pour effectuer une histologie à la fin de l'expérience.

Après traitement par les actifs à tester et le produit de référence (vitamine C), les épidermes ont été lavés et la caféine a été déposée à la surface de chaque épiderme à raison de 100 µl à 2 µCi/ml (0.04 mM) de caféine radioactive et 0.35 mM de caféine froide, ce qui correspond à environ 500 000 cpm totaux.

On a mesuré le % d'activité de caféine après 2h d'application.

Les résultats obtenus sont répertoriés dans le tableau suivant :

| TRAITEMENT | CONCENTRATION | % activité par rapport au témoin | Diminution du passage de la caféine après 2h d'application (en %) |
|---|---|---|---|
| Vitamine C | 200µg/ml | 59 | - 41% |
| Hydroxyapatite* | 50µg/ml | 64 | - 36% |
| Hydroxyapatite | 5µg/ml | 92 (NS) | NS |
| Hydroxyapatite + Calcium Pantetheine Sulfonate | 5µg/ml et 0.003 % | 60 | - 40% |

| | | | |
|---|---|---|---|
| *(NS = non significatif)* *= Hydroxysomes™ commercialisés par LSC | | | |

En parallèle et selon les mêmes conditions de test, nous avons évalué l'effet du calcium D-pantéthéine sulfonate seul à 0,03% et 0,003%, sur la fonction barrière de la peau. Après 2h d'application, seule la plus forte concentration (0,03%) a montré une diminution significative de la diffusion de caféine (-24% par rapport au témoin). Le résultat n'était pas significatif pour la concentration de 0,003%.

Les résultats obtenus montrent que la vitamine C (200µg/ml), utilisée comme molécule de référence, ralentit significativement le passage de la caféine (-41% par rapport au témoin, p < 0.01). Ces résultats ont permis de valider l'essai.

L'hydroxyapatite testée seule à 50µg/ml ralentit la diffusion de la caféine (- 36 % de passage, p<0.05). A 5µg/ml, cet actif ne montre pas d'effet.

Le mélange hydroxyapatite 5µg/ml et Calcium Pantetheine Sulfonate 0,003%, diminue significativement le passage de la caféine (- 40 %, p<0.05).

Ces résultats montrent que l'effet de l'hydroxyapatite semble potentialisé par le Calcium Pantetheine Sulfonate, qui à 0,003% n'avait pas d'effet à lui seul sur la fonction barrière cutanée, et que cet effet est voisin de celui observé avec la Vitamine C.

Les résultats obtenus pour cette association d'actifs traduisent une amélioration et/ou un renforcement de la fonction barrière épidermique.

### Exemple 2 : Formulation

**Emulsion huile-dans-eau**

| | |
|---|---|
| Calcium pantetheine sulfonate* | 0,10% |
| Hydroxyapatite** | 0,50% |
| Huiles | 6,00% |
| Alcool stéarylique | 0,50% |
| Filtres UV | 10,50% |
| Cyclopentasiloxane | 7,50% |
| Triéthanolamine | 1,60% |
| Glycérine | 7,00% |
| Emulsionnants | 5,80% |
| Parfums | 0,30% |
| Conservateurs | 1,20% |
| Eau | qsp 100% |

| | |
|---|---|
| *= bis(N-pantothenyl-beta-aminoethyl) thiosulfate de calcium en solution aqueuse à 70% commercialisé par SOGO PHARMACEUTICAL **= Hydroxysomes™ commercialisés par LSC | |

Après application sur la peau, celle-ci est visiblement plus souple et hydratée.

## Revendications

1. Composition comprenant, dans un milieu physiologiquement acceptable, au moins de l'hydroxyapatite au moins en partie sous forme libre et au moins le pantetheine sulfonate de calcium.

2. Composition selon la revendication 1, **caractérisée en ce que** l'hydroxyapatite est présente sous forme de particules ayant une taille moyenne en nombre allant de 0,1 à 50µm.

3. Composition selon l'une des revendications 1 ou 2, **caractérisée en ce que** l'hydroxyapatite est présente sous forme de particules ayant une taille moyenne en nombre allant de 1 à 10µm.

4. Composition selon l'une des revendications 1 à 3, **caractérisée en ce que** l'hydroxyapatite est sous forme de particules comprenant au moins 99% en poids d'hydroxyapatite par rapport au poids total des particules.

5. Composition selon l'une quelconque des revendications précédentes, **caractérisée en ce que** l'hydroxyapatite est présente dans la composition en une quantité allant de 0,01 à 5% en poids par rapport au poids total de la composition.

6. Composition selon l'une quelconque des revendications précédentes, **caractérisée en ce que** le pantetheine sulfonate de calcium est présent dans la composition en une quantité allant de 0,01 à 5% en poids par rapport au poids total de la composition.

7. Composition selon l'une quelconque des revendications précédentes, **caractérisée en ce que** l'hydroxyapatite et le pantetheine sulfonate de calcium sont présents dans la composition dans un rapport de concentration allant de 10 : 1 à 1 : 5.

8. Composition selon l'une quelconque des revendications précédentes, **caractérisée en ce qu'**elle comprend en outre au moins un agent choisi parmi les agents desquamants et/ou hydratants ; les agents stimulant la synthèse de macromolécules du derme et/ou prévenant leur dégradation ; les agents stimulant la prolifération des fibroblastes ou des kératinocytes et/ou la différenciation des kératinocytes ; les agents favorisant la synthèse des lipides épidermiques ; les lipides épidermiques; les agents anti-oxydants et anti-radicalairess et leurs mélanges.

9. Composition selon l'une quelconque des revendications précédentes, **caractérisée en ce qu'**elle comprend en outre au moins un filtre UV.

10. Composition selon l'une quelconque des revendications précédentes, **caractérisée en ce qu'**elle est sous une forme adaptée à une application topique sur la peau et/ou les semi-muqueuses.

11. Composition selon l'une des revendications 1 à 9, **caractérisée en ce qu'**elle est sous une forme adaptée à une administration par voie orale.

12. Procédé non-thérapeutique cosmétique de soin de la peau et/ou des semi-muqueuses comprenant l'administration par voie orale et l'application topique sur la peau et/ou les lèvres, *via* au moins deux compositions, de l'association d'au moins de l'hydroxyapatite au moins en partie sous forme libre et d'au moins le pantetheine sulfonate de calcium.

13. Procédé non-thérapeutique cosmétique de soin de la peau et/ou des semi-muqueuses comprenant l'administration par voie orale ou l'application topique sur la peau et/ou les semi-muqueuses d'une composition telle que définie dans l'une quelconque des revendications 1 à 11.

14. Procédé non-thérapeutique selon l'une des revendications 12 ou 13, **caractérisé en ce qu'**il vise à améliorer l'hydratation et/ou le confort de la peau et/ou renforcer la protection de la peau et/ou des semi-muqueuses contre les agressions extérieures.

15. Procédé non-thérapeutique selon l'une des revendications 12 ou 13, **caractérisé en ce que** qu'il vise à prévenir et/ou diminuer la formation des crevasses ou gerçures sur la peau et/ou les semi-muqueuses.

16. Procédé non-thérapeutique selon l'une des revendications 12 à 15, **caractérisée en ce que** la composition est administrée et/ou appliquée sur des sujets ayant une peau mature, voire très mature.

17. Utilisation cosmétique non-thérapeutique de l'association d'au moins de l'hydroxyapatite au moins en partie sous forme libre et d'au moins le pantetheine sulfonate de calcium, comme association destinée à améliorer et/ou renforcer la cohésion cellulaire et/ou l'intégrité de la peau et/ou des semi-muqueuses et/ou améliorer et/ou renforcer la fonction barrière de la peau et/ou des semi-muqueuses.

18. Utilisation non-thérapeutique selon la revendication 17, **caractérisée en ce que** ladite association est destinée à prévenir et/ou diminuer la fragilisation de la peau et/ou des semi-muqueuses et/ou protéger la peau et/ou les semi-muqueuses contre les déperditions en eau et/ou les agressions extérieures.

19. Utilisation non-thérapeutique selon l'une des revendications 17 ou 18, **caractérisée en ce que** l'hydroxyapatite et le pantetheine sulfonate de calcium sont conditionnés dans une même composition.

20. Utilisation non-thérapeutique selon l'une des revendications 17 ou 18, **caractérisée en ce que** l'hydroxyapatite et le pantetheine sulfonate de calcium sont conditionnés dans des compositions distinctes.

## Patentansprüche

1. Zusammensetzung, die in einem physiologisch annehmbaren Medium mindestens Hydroxyapatit, zumindest zum Teil in freier Form, und mindestens Calciumpantetheinsulfonat umfasst.

2. Zusammensetzung nach Anspruch 1, **dadurch gekennzeichnet, dass** das Hydroxyapatit in Form von Partikeln mit einer Partikelgröße im Zahlenmittel von 0,1-50 µm vorliegt.

3. Zusammensetzung nach einem der Ansprüche 1 bis 2, **dadurch gekennzeichnet, dass** das Hydroxyapatit in Form von Partikeln mit einer Partikelgröße im Zahlenmittel von 1 bis 10 µm vorliegt.

4. Zusammensetzung nach einem der Ansprüche 1 bis 3, **dadurch gekennzeichnet, dass** das Hydroxyapatit in Form von Partikeln vorliegt, die mindestens 99 Gew.-% Hydroxyapatit, bezogen auf das Gesamtgewicht der Partikel, umfassen.

5. Zusammensetzung nach einem der vorhergehenden Ansprüche, **dadurch gekennzeichnet, dass** das Hydroxyapatit in der Zusammensetzung in einer Menge von 0,01 Gew.-% bis 5 Gew.-%, bezogen auf das Gesamtgewicht der Zusammensetzung, vorliegt.

6. Zusammensetzung nach einem der vorhergehenden Ansprüche, **dadurch gekennzeichnet, dass** das Calciumpantetheinsulfonat in der Zusammensetzung in einer Menge von 0,01 Gew.-% bis 5 Gew.-%, bezogen auf das Gesamtgewicht der Zusammensetzung, vorliegt.

7. Zusammensetzung nach einem der vorhergehenden Ansprüche, dadurch gekennzeichet, dass das Hydroxyapatit und das Calciumpantetheinsulfonat in der Zusammensetzung in einem Konzentrationsverhältnis von 10:1 bis 1:5 vorhanden sind.

8. Zusammensetzung nach einem der vorhergehenden Ansprüche, **dadurch gekennzeichnet, dass** sie außerdem mindestens ein aus Schäl- und/oder Feuchthaltemitteln; die Synthese von Makromolekülen der Dermis stimulierenden und/oder deren Abbau verhindernden Mitteln; die Proliferation von Fibroblasten oder Keratinozyten und/oder die Differenzierung von Keratinozyten stimulierenden Mitteln; die Synthese epidermaler Lipide fördernden Mitteln; epidermalen Lipiden; Antioxidantien und Radikalfängern und deren Gemischen ausgewähltes Mittel umfasst.

9. Zusammensetzung nach einem der vorhergehenden Ansprüche, **dadurch gekennzeichnet, dass** sie außerdem mindestens einen UV-Filter umfasst.

10. Zusammensetzung nach einem der vorhergehenden Ansprüche, **dadurch gekennzeichnet, dass** sie in einer an die topische Applikation auf die Haut und/oder die Halbschleimhäute angepassten Form vorliegt.

11. Zusammensetzung nach einem der Ansprüche 1 bis 9, **dadurch gekennzeichnet, dass** sie in einer an die orale Verabreichung angepassten Form vorliegt.

12. Nichttherapeutisches kosmetisches Verfahren zur Pflege der Haut und/oder der Halbschleimhäute, umfassend die Verabreichung auf oralem Weg oder die topische Applikation auf die Haut und/oder die Lippen, über mindestens zwei Zusammensetzungen, der Vereinigung von mindestens Hydroxyapatit, zumindest zum Teil in freier Form, und mindestens Calciumpantetheinsulfonat.

13. Nichttherapeutisches kosmetisches Verfahren zur Pflege der Haut und/oder der Halbschleimhäute, umfassend die Verabreichung auf oralem Weg oder die topische Applikation auf die Haut und/oder die Lippen einer Zusammensetzung nach einem der Ansprüche 1 bis 11.

14. Nichttherapeutisches Verfahren nach einem der Ansprüche 12 oder 13, **dadurch gekennzeichnet, dass** es auf die Verbesserung der Feuchtigkeitsversorgung und/oder des Wohlbefindens der Haut und/oder auf die Verstärkung des Schutzes der Haut und/oder der Halbschleimhäute gegen äußere Angriffe abzielt.

15. Nichttherapeutisches Verfahren nach einem der Ansprüche 12 oder 13, **dadurch gekennzeichnet, dass** es auf die Vorbeugung und/oder Verringerung der Bildung von Rissen oder Schrunden auf der Haut und/oder den Halbschleimhäuten abzielt.

16. Nichttherapeutisches Verfahren nach einem der Ansprüche 12 bis 15, **dadurch gekennzeichnet, dass** die Zusammensetzung an Individuen verabreicht und/oder appliziert wird, die eine reife oder sogar sehr Haut haben.

17. Nichttherapeutische kosmetische Verwendung der Vereinigung von mindestens Hydroxyapatit, zumindest zum Teil in freier Form, und mindestens Calciumpantetheinsulfonat als Vereinigung zur Verbesserung und/oder Verstärkung der Zellkohäsion und/oder der Unversehrtheit der Haut und/oder der Halbschleimhäute und/oder zur Verbesserung und/oder Verstärkung der Barrierefunktion der Haut und/oder der Halbschleimhäute.

18. Nichttherapeutische Verwendung nach Anspruch 17, **dadurch gekennzeichnet, dass** die Vereinigung zur Vorbeugung und/oder Verringerung der Sprödigkeit der Haut und/oder der Halbschleimhäute und/oder zum Schutz der Haut und/oder der Halbschleimhäute gegen Wasserverluste und/oder äußere Angriffe bestimmt ist.

19. Nichttherapeutische Verwendung nach einem der Ansprüche 17 oder 18, **dadurch gekennzeichnet, dass** das Hydroxyapatit und das Calciumpantetheinsulfonat in ein und derselben Zusammensetzung abgepackt sind.

20. Nichttherapeutische Verwendung nach einem der Ansprüche 17 oder 18, **dadurch gekennzeichnet, dass** das Hydroxyapatit und das Calciumpantetheinsulfonat in getrennten Zusammensetzungen abgepackt sind.

## Claims

1. Composition comprising, in a physiologically acceptable medium, at least hydroxyapatite that is at least partially in free form and at least calcium pantetheine sulfonate.

2. Composition according to Claim 1, **characterized in that** the hydroxyapatite is present in the form of particles with a numerical mean size ranging from 0.1 to 50 µm.

3. Composition according to either of Claims 1 and 2, **characterized in that** the hydroxyapatite is present in the form of particles with a numerical mean size ranging from 1 to 10 µm.

4. Composition according to one of Claims 1 to 3, **characterized in that** the hydroxyapatite is in the form of particles comprising at least 99% by weight of hydroxyapatite relative to the total weight of the particles.

5. Composition according to any one of the preceding claims, **characterized in that** the hydroxyapatite is present in the composition in an amount ranging from 0.01% to 5% by weight relative to the total weight of the composition.

6. Composition according to any one of the preceding claims, **characterized in that** the calcium pantetheine sulfonate is present in the composition in an amount ranging from 0.01% to 5% by weight relative to the total weight of the composition.

7. Composition according to any one of the preceding claims, **characterized in that** the hydroxyapatite and the calcium pantetheine sulfonate are present in the composition in a concentration ratio ranging from 10:1 to 1:5.

8. Composition according to any one of the preceding claims, **characterized in that** it also comprises at least one agent chosen from desquamating agents and/or moisturizers; agents for stimulating the synthesis of dermal macromolecules and/or for preventing their degradation; agents for stimulating fibroblast or keratinocyte proliferation and/or keratinocyte differentiation; agents for promoting the synthesis of epidermal lipids; epidermal lipids; antioxidants and free-radical scavengers, and mixtures thereof.

9. Composition according to any one of the preceding claims, **characterized in that** it also comprises at least one UV-screening agent.

10. Composition according to any one of the preceding claims, **characterized in that** it is in a form suitable for topical application to the skin and/or semi-mucous membranes.

11. Composition according to one of Claims 1 to 9, **characterized in that** it is in a form suitable for oral administration.

12. Non therapeutic cosmetic process for caring for the skin and/or semi-mucous membranes, comprising the oral administration and the topical application to the skin and/or the lips via at least two compositions, of the combination of at least hydroxyapatite that is at least partially in free form and of at least calcium pantetheine sulfonate.

13. Non therapeutic cosmetic process for caring for the skin and/or semi-mucous membranes, comprising the oral administration or the topical application to the skin and/or semi-mucous membranes of a composition as defined in any one of Claims 1 to 11.

14. Non therapeutic process according to either of Claims 12 and 13, **characterized in that** it is directed towards improving the moisturization and/or comfort of the skin and/or towards reinforcing the protection of the skin and/or semi-mucous membranes against external attack.

15. Non therapeutic process according to either of Claims 12 and 13, **characterized in that** it is directed towards preventing and/or reducing the formation of cracking or chapping on the skin and/or semi-mucous membranes.

16. Non therapeutic process according to one of Claims 12 to 15, **characterized in that** the composition is administered and/or applied to individuals with mature or even very mature skin.

17. Non therapeutic cosmetic use of the combination of at least hydroxyapatite that is at least partially in free form and of at least calcium pantetheine sulfonate, as a combination for improving and/or reinforcing the cellular cohesion and/or the integrity of the skin and/or semi-mucous membranes and/or for improving and/or reinforcing the barrier function of the skin and/or semi-mucous membranes.

18. Non therapeutic use according to Claim 17, **characterized in that** the said combination is intended for preventing and/or reducing the embrittlement of the skin and/or semi-mucous membranes and/or for protecting the skin and/or semi-mucous membranes against water loss and/or external attack.

19. Non therapeutic use according to either of Claims 17 and 18, **characterized in that** the hydroxyapatite and the calcium pantetheine sulfonate are conditioned in the same composition.

20. Non therapeutic use according to either of Claims 17 and 18, **characterized in that** the hydroxyapatite and the calcium pantetheine sulfonate are conditioned in separate compositions.
